# EUROPEAN PATENT APPLICATION

(11) **EP 4 667 453 A1**
(43) Date of publication of application: **24.12.2025**
(21) Application number: 23922958.6
(22) Date of filing: 25.12.2023
(51) Int. Cl.: C07C 227/16, C07C 229/36

(54) **METHOD FOR PRODUCING TRYPTOPHAN DECOMPOSITION PRODUCT, METHOD FOR PRODUCING FORMYLKYNURENINE, METHOD FOR PRODUCING KYNURENINE, METHOD FOR PRODUCING TRYPTOPHANRADICALS-CONTAINING AQUEOUS SOLUTION, AND BACTERICIDAL AQUEOUS SOLUTION**

(30) Priority: 13.02.2023 JP 2023020290
(71) Applicant: NATIONAL UNIVERSITY CORPORATION TOKAI NATIONAL HIGHER EDUCATION AND RESEARCH SYSTEM, Nagoya-shi, Aichi 464-8601 (JP); Meijo University, Nagoya-shi, Aichi 468-8502 (JP)
(72) Inventor: HORI Masaru, Nagoya-shi, Aichi 464-8601 (JP); HASHIZUME Hiroshi, Nagoya-shi, Aichi 464-8601 (JP); ISHIKAWA Kenji, Nagoya-shi, Aichi 464-8601 (JP); IWATA Naoyuki, Nagoya-shi, Aichi 464-8601 (JP); ITO Masafumi, Nagoya-shi, Aichi 468-8502 (JP); NISHIKAWA Yasuhiro, Nagoya-shi, Aichi 468-8502 (JP); ARAKI Shota, Nagoya-shi, Aichi 468-8502 (JP)
(74) Representative: Kramer Barske Schmidtchen Patentanwälte PartG mbB
(86) International application number: PCT/JP2023/046343
(87) International publication number: WO 2024/171625

(57) **Abstract**

By irradiating a Trp-containing aqueous solution with oxygen radicals, a precursor including at least one of Trp• and Trp peroxide is generated from Trp, and a Trp decomposition product including at least one of FKYN and KYN is generated from the precursor, thereby obtaining an oxygen radical-irradiated aqueous solution. Subsequently, the Trp decomposition product such as FKYN or KYN is separated from the oxygen radical-irradiated aqueous solution. By irradiating the Trp-containing aqueous solution with oxygen radicals, a Trp•-containing aqueous solution that contains Trp• is obtained, which is used as a bactericidal aqueous solution.

## Description

### CROSS-REFERENCE TO RELATED APPLICATIONS

The present application is based on Japanese Application No. 2023-20290 filed on February 13, 2023, the contents of which are incorporated herein by reference.

### TECHNICAL FIELD

The present disclosure relates to a method for producing a tryptophan decomposition product, a method for producing formylkynurenine, a method for producing kynurenine, a method for producing a tryptophan radical-containing aqueous solution, and a bactericidal aqueous solution.

### BACKGROUND ART

Plasma technology is applied to the fields of electricity, chemistry, and materials. In addition to charged particles such as electrons and ions, neutral particles such as atoms and molecules, ultraviolet photons, and the like are generated inside plasmas. Among these products generated inside plasmas, particles (including atoms, molecules, ions) having unpaired electrons are generally referred to as radicals.

Prior Patent Literature 1 describes that by irradiating an aqueous solution containing a cyclic organic compound having at least one of a benzene ring, a pyrrole ring, and a pyridine ring with oxygen radicals, an oxygen radical-activated aqueous solution capable of exhibiting a bactericidal effect while exhibiting substantially neutrality is obtained. This Patent Literature 1 describes tryptophan as an example of a cyclic organic compound having a benzene ring and a pyrrole ring.

Tryptophan is a substance widely known as one of the nine essential amino acids in humans. In addition, examples of tryptophan decomposition products include formylkynurenine and kynurenine.

### PRIOR ART LITERATURE

### Patent Literature

Patent Literature 1: JP 2020-33294 A

### SUMMARY OF INVENTION

### PROBLEMS TO BE SOLVED BY INVENTION

Formylkynurenine and kynurenine are extremely expensive reagents. It would be extremely useful if tryptophan decomposition products such as formylkynurenine and kynurenine could be produced relatively simply.

The above-described Patent Literature 1 describes an activated aqueous solution obtained by irradiating a tryptophan solution with oxygen radicals, but there is no disclosure or suggestion at all regarding any components in the activated aqueous solution. In addition, a bactericidal substance in the above-described activated aqueous solution is also unknown.

The present disclosure has been made in view of such problems, and is directed to providing a method for producing a tryptophan decomposition product that can relatively simply produce a tryptophan decomposition product, a method for producing formylkynurenine that can relatively simply produce formylkynurenine, a method for producing kynurenine that can relatively simply produce kynurenine, a method for producing a tryptophan radical-containing aqueous solution usable as a bactericidal aqueous solution, and a bactericidal aqueous solution.

### MEANS FOR SOLVING PROBLEM

One aspect of the present disclosure is a method for producing a tryptophan decomposition product, the method including:
obtaining an oxygen radical-irradiated aqueous solution that contains a tryptophan decomposition product by irradiating a tryptophan-containing aqueous solution that contains tryptophan with oxygen radicals so as to generate a precursor including at least one of a tryptophan radical and tryptophan peroxide from the tryptophan and thereafter generate a tryptophan decomposition product including at least one of formylkynurenine and kynurenine from the precursor; and
separating the tryptophan decomposition product from the oxygen radical-irradiated aqueous solution.

Another aspect of the present disclosure is a method for producing formylkynurenine, the method including:
obtaining an oxygen radical-irradiated aqueous solution that contains formylkynurenine by irradiating a tryptophan-containing aqueous solution that contains tryptophan with oxygen radicals so as to generate a precursor including at least one of a tryptophan radical and tryptophan peroxide from the tryptophan and thereafter generate the formylkynurenine from the precursor; and
separating the formylkynurenine from the oxygen radical-irradiated aqueous solution.

Still another aspect of the present disclosure is a method for producing kynurenine, the method including:
obtaining an oxygen radical-irradiated aqueous solution that contains kynurenine by irradiating a tryptophan-containing aqueous solution that contains tryptophan with oxygen radicals so as to generate a precursor including at least one of a tryptophan radical and tryptophan peroxide from the tryptophan and thereafter generate the kynurenine from the precursor; and
separating the kynurenine from the oxygen radical-irradiated aqueous solution.

Still another aspect of the present disclosure is a method for producing a tryptophan radical-containing aqueous solution, wherein a tryptophan-containing aqueous solution that contains tryptophan is irradiated with oxygen radicals to generate tryptophan radicals from the tryptophan to thereby generate a tryptophan radical-containing aqueous solution that contains the tryptophan radicals.

Still another aspect of the present disclosure is a bactericidal aqueous solution having a bactericidal effect and including a tryptophan radical-containing aqueous solution that contains tryptophan radicals.

### EFFECTS OF INVENTION

The above-described method for producing a tryptophan decomposition product has the above-described configuration. Therefore, according to the above-described method for producing a tryptophan decomposition product, by irradiating a tryptophan-containing aqueous solution that contains tryptophan with oxygen radicals, the tryptophan decomposition product can be produced from tryptophan simply in a relatively short time.

The above-described method for producing formylkynurenine has the above-described configuration. Therefore, according to the above-described method for producing formylkynurenine, by irradiating a tryptophan-containing aqueous solution that contains tryptophan with oxygen radicals, formylkynurenine, which is one of the tryptophan decomposition products, can be produced from tryptophan simply in a relatively short time.

The above-described method for producing kynurenine has the above-described configuration. Therefore, according to the above-described method for producing kynurenine, by irradiating a tryptophan-containing aqueous solution that contains tryptophan with oxygen radicals, kynurenine, which is one of the tryptophan decomposition products, can be produced from tryptophan simply in a relatively short time.

The above-described method for producing a tryptophan radical-containing aqueous solution has the above-described configuration. Therefore, according to the above-described method for producing a tryptophan radical-containing aqueous solution, a tryptophan radical-containing aqueous solution that contains a tryptophan radical as a bactericidal substance and is suitably usable as a bactericidal aqueous solution can be produced.

The above-described bactericidal aqueous solution has the above-described configuration. Therefore, the above-described bactericidal aqueous solution can exhibit a bactericidal effect due to the tryptophan radical, which is a bactericidal substance.

### BRIEF DESCRIPTION OF DRAWINGS

Fig. 1 is an explanatory diagram schematically illustrating a structure of a radical source applicable to irradiation with oxygen radicals in a method for producing a tryptophan decomposition product, a method for producing formylkynurenine, and a method for producing kynurenine according to a first embodiment, as well as a method for producing a tryptophan radical-containing aqueous solution according to a second embodiment.
Fig. 2 is an explanatory diagram showing a generation pathway of formylkynurenine, kynurenine, a tryptophan radical, and the like generated when a tryptophan-containing aqueous solution is irradiated with oxygen radicals in the method for producing a tryptophan decomposition product, the method for producing formylkynurenine, and the method for producing kynurenine according to the first embodiment, as well as the method for producing a tryptophan radical-containing aqueous solution according to the second embodiment.
Fig. 3 is a diagram showing gradient conditions of a mobile phase during component fractionation using a high performance liquid chromatograph (hereinafter sometimes referred to as HPLC) in Experimental Example 1.
Fig. 4 is a diagram showing the results of HPLC in Experimental Example 1.
Fig. 5 is a diagram showing the test results for confirming the bactericidal effect of a fractionated product obtained by HPLC in Experimental Example 1.
Fig. 6 is a diagram showing gradient conditions of a mobile phase during component analysis using LC-MS in Experimental Example 2, and gradient conditions of a mobile phase during component analysis using HPLC in Experimental Example 4.
Fig. 7 provides diagrams showing (a) an LC-MS chromatogram and (b) an MS spectrum for a component P1 in Experimental Example 2.
Fig. 8 provides diagrams showing (a) an LC-MS chromatogram and (b) an MS spectrum for a component P2 in Experimental Example 2.
Fig. 9 provides diagrams showing (a) a ¹H-NMR spectrum and (b) a ¹³C-NMR spectrum for the component P1 and FKYN (standard substance) in Experimental Example 2.
Fig. 10 provides diagrams showing (a) a ¹H-NMR spectrum and (b) a ¹³C-NMR spectrum for the component P2 and KYN (standard substance) in Experimental Example 2.
Fig. 11 is a diagram showing the results of ESR measurement in Experimental Example 3.
Fig. 12 provides explanatory diagrams regarding (a) a reaction between DBNBS and Trp•, and (b) the Fenton reaction and a reaction between Trp and OH• in Experimental Example 3.
Fig. 13 is a diagram showing chromatograms by HPLC analysis in Experimental Example 4.
Fig. 14 is a diagram collectively showing the relationship between the irradiation time of oxygen radicals and area values of the component P1 and the component P2 calculated from the chromatograms of Fig. 13 in Experimental Example 4.
Fig. 15 is a diagram showing UV absorption spectra of the component P1 in Experimental Example 4.
Fig. 16 is a diagram showing UV absorption spectra of the component P2 in Experimental Example 4.
Fig. 17 provides diagrams showing quantitative results of FKYN in an oxygen radical-irradiated aqueous solution using an FKYN standard reagent solution in Experimental Example 5.
Fig. 18 provides diagrams showing quantitative results of KYN in an oxygen radical-irradiated aqueous solution using a KYN standard reagent solution in Experimental Example 5.
Fig. 19 is a diagram collectively showing the relationship between the irradiation time of oxygen radicals and the amounts of fractionated FKYN and KYN in Experimental Example 5.
Fig. 20 is a diagram showing chromatograms obtained by HPLC analysis of aqueous solutions in Experimental Example 6.
Fig. 21 is a diagram showing UV absorption spectra of tryptophan (Trp) in Experimental Example 6.
Fig. 22 is a diagram showing chromatograms obtained by HPLC analysis for plasma-irradiated aqueous solutions with different plasma irradiation times in Experimental Example 6.
Fig. 23 is a diagram showing chromatograms obtained by HPLC analysis for oxygen radical-irradiated aqueous solutions with different pH values and an oxygen radical-unirradiated aqueous solution in Experimental Example 7.
Fig. 24 is a diagram showing UV absorption spectra of the component P1 (FKYN) obtained for the oxygen radical-irradiated aqueous solutions with different pH values in Experimental Example 7.
Fig. 25 is a diagram showing UV absorption spectra of the component P2 (KYN) obtained for the oxygen radical-irradiated aqueous solutions with different pH values in Experimental Example 7.
Fig. 26 is a diagram showing UV absorption spectra of tryptophan (Trp) obtained for the oxygen radical-irradiated aqueous solutions with different pH values in Experimental Example 7.
Fig. 27 is a diagram showing area values of the component P1 (FKYN) and the component P2 (KYN) calculated from the chromatograms in Fig. 23 by HPLC analysis for the oxygen radical-irradiated aqueous solutions with different pH values and the oxygen radical-unirradiated aqueous solution in Experimental Example 7.

### MODE FOR CARRYING OUT INVENTION

Hereinafter, a method for producing a tryptophan decomposition product, a method for producing formylkynurenine, a method for producing kynurenine, a method for producing a tryptophan radical-containing aqueous solution, and a bactericidal aqueous solution of present embodiments will be described. The method for producing a tryptophan decomposition product, the method for producing formylkynurenine, the method for producing kynurenine, the method for producing a tryptophan radical-containing aqueous solution, and the bactericidal aqueous solution of the present embodiments are not limited by the following examples.

### (First embodiment)

A method for producing a tryptophan decomposition product, a method for producing formylkynurenine, and a method for producing kynurenine according to a first embodiment will be described.

The method for producing a tryptophan decomposition product of the present embodiment is a method for producing a tryptophan decomposition product, the method including:
obtaining an oxygen radical-irradiated aqueous solution that contains a tryptophan decomposition product by irradiating a tryptophan-containing aqueous solution that contains tryptophan with oxygen radicals so as to generate a precursor including at least one of a tryptophan radical and tryptophan peroxide from the tryptophan and thereafter generate a tryptophan decomposition product including at least one of formylkynurenine and kynurenine from the precursor; and
separating the tryptophan decomposition product from the oxygen radical-irradiated aqueous solution to thereby produce the tryptophan decomposition product.

In addition, the method for producing formylkynurenine of the present embodiment is a method for producing formylkynurenine, the method including:
obtaining an oxygen radical-irradiated aqueous solution that contains formylkynurenine by irradiating a tryptophan-containing aqueous solution that contains tryptophan with oxygen radicals so as to generate a precursor including at least one of a tryptophan radical and tryptophan peroxide from the tryptophan and thereafter generate the formylkynurenine from the precursor; and
separating the formylkynurenine from the oxygen radical-irradiated aqueous solution to thereby produce the formylkynurenine.

In addition, the method for producing kynurenine of the present embodiment is a method for producing kynurenine, the method including:
obtaining an oxygen radical-irradiated aqueous solution that contains kynurenine by irradiating a tryptophan-containing aqueous solution that contains tryptophan with oxygen radicals so as to generate a precursor including at least one of a tryptophan radical and tryptophan peroxide from the tryptophan and thereafter generate the kynurenine from the precursor; and
separating the kynurenine from the oxygen radical-irradiated aqueous solutionto thereby produce the kynurenine. Hereinafter, these will be described in detail.

In the method for producing a tryptophan decomposition product of the present embodiment, a precursor including at least one of a tryptophan radical and tryptophan peroxide is generated from tryptophan by irradiating a tryptophan-containing aqueous solution that contains the tryptophan with oxygen radicals, and a tryptophan decomposition product including at least one of formylkynurenine and kynurenine is further generated from the precursor, thereby obtaining an oxygen radical-irradiated aqueous solution containing the tryptophan decomposition product.

In addition, in the method for producing formylkynurenine of the present embodiment, a precursor including at least one of a tryptophan radical and tryptophan peroxide is generated from tryptophan by irradiating a tryptophan-containing aqueous solution that contains the tryptophan with oxygen radicals, and formylkynurenine is further generated from the precursor, thereby obtaining an oxygen radical-irradiated aqueous solution containing the formylkynurenine.

In addition, in the method for producing kynurenine of the present embodiment, a precursor including at least one of a tryptophan radical and tryptophan peroxide is generated from tryptophan by irradiating a tryptophan-containing aqueous solution that contains the tryptophan with oxygen radicals, and kynurenine is further generated from the precursor, thereby obtaining an oxygen radical-irradiated aqueous solution containing the kynurenine.

In the present specification and drawings, tryptophan may be appropriately denoted as Trp, tryptophan radical as Trp•, tryptophan peroxide as Trp peroxide, formylkynurenine as FKYN, and kynurenine as KYN.

The tryptophan-containing aqueous solution is an aqueous solution containing tryptophan. Tryptophan may be either L-type tryptophan or D-type tryptophan. Tryptophan is preferably L-tryptophan (which may be appropriately denoted as L-Trp in the present specification and drawings) from the viewpoint of abundance ratio in nature, price suppression, and the like. From the viewpoint of solubility and the like, the tryptophan-containing aqueous solution can contain tryptophan preferably at a concentration of 100 mM or less, more preferably 90 mM or less, and still more preferably 80 mM or less, with respect to a solubility limit of water as a solvent. In addition, the tryptophan-containing aqueous solution can contain tryptophan preferably at a concentration of 10 mM or more, more preferably 20 mM or more, and still more preferably 30 mM or more, from the viewpoint of yield and the like. The above-described M means mol/L.

Oxygen radicals are neutral particles possessing unpaired electrons. In a strict sense, a singlet oxygen atom (O(¹D)) and a singlet oxygen molecule (O₂(¹Δ_{g})) do not have unpaired electrons; however, in the present disclosure, an oxygen radical is defined to include not only a triplet oxygen atom (O(³Pⱼ)) but also one or more of a singlet oxygen atom (O(¹D)), a singlet oxygen molecule (O₂(¹Δ_{g})), and ozone (O₃).

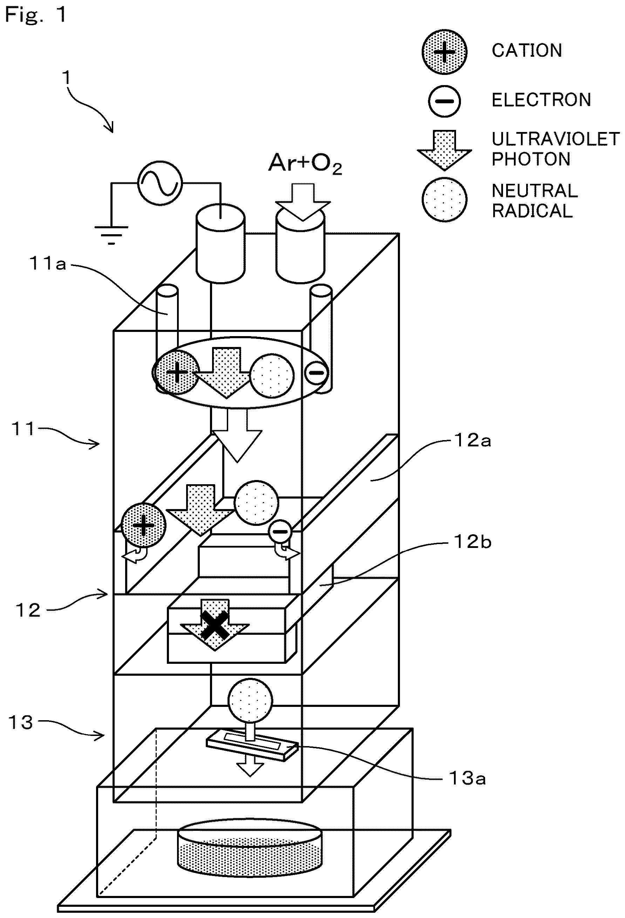

As a radical source for the irradiation of oxygen radicals, for example, a non-equilibrium atmospheric pressure radical source capable of extracting neutral oxygen radicals from plasma under atmospheric pressure and irradiating therewith can be used. Specifically, as illustrated in Fig. 1, a radical source 1 has a three-stage structure consisting of a discharge unit 11, an intermediate unit 12, and an irradiation unit 13. The discharge unit 11 is supplied with an inert gas (for example, a rare gas such as argon) and an oxygen gas (O₂) for radical generation. Then, plasma is generated by discharge in the space between an opposing electrode pair 11a. The plasma gas contains positive ions, electrons, neutral radicals, ultraviolet photons, and the like. In the intermediate unit 12, positive ions and electrons in the plasma are removed by a grounded electrode 12a. In addition, ultraviolet photons are removed by a curved path 12b located below the grounded electrode 12a. The irradiation unit 13 emits neutral radicals extracted from the plasma, specifically neutral oxygen radicals, from an irradiation port 13a. The irradiation port 13a can, for example, have a single slit, and the slit can be configured with an opening that opens in a shape of, for example, 16 mm in length and 0.5 mm in width. Such a radical source 1 can selectively emit neutral oxygen radicals and substantially does not emit positive ions, electrons, or photons such as ultraviolet photons.

For the details of the above-described radical source and its peripheral equipment, for example, those described in JP 2020-33294 A or the like or "Tough Plasma" manufactured by FUJI CORPORATION and its improved products can be suitably used. In addition, the technology described in the above-described publication can be incorporated herein as needed.

The plasma density in a plasma generation region of the discharge unit can be, from the viewpoint of processing speed and the like, preferably 10¹⁴ cm⁻³ or more and 10¹⁷ cm⁻³ or less, more preferably 10¹⁵ cm⁻³ or more and 10¹⁷ cm⁻³ or less, and still more preferably 10¹⁶ cm⁻³ or more and 10¹⁷ cm⁻³ or less.

The density of triplet oxygen atoms (O(³Pⱼ)) during oxygen radical irradiation can be, from the viewpoint of processing speed and the like, preferably 10¹³ cm⁻³ or more and 10¹⁶ cm⁻³ or less, more preferably 10¹⁴ cm⁻³ or more and 10¹⁶ cm⁻³ or less, and still more preferably 10¹⁵ cm⁻³ or more and 10¹⁶ cm⁻³ or less. When a mixed gas of argon and oxygen gas is used as a plasma generation gas supplied to the discharge unit of the above-described radical source, the density of triplet oxygen atoms (O(³Pⱼ)) can reach a maximum of approximately 10¹⁴ to 10¹⁶ cm⁻³ when the volume percentage of oxygen gas, calculated as a percentage based on the oxygen gas flow rate to the total gas flow rate of argon and oxygen gas, is about 0.5% to 0.7%.

The density of triplet oxygen atoms (O(³Pⱼ)) tends to decrease exponentially as an irradiation distance, which is a distance from the irradiation port to a target, increases. The irradiation distance during oxygen radical irradiation can be, from the viewpoints of radical lifespan and suppression of back-diffusion from the liquid surface, preferably 3 mm or longer and 20 mm or shorter, more preferably 3 mm or longer and 15 mm or shorter, and still more preferably from 3 mm ore longer and 10 mm or shorter.

The irradiation of oxygen radicals can be, for example, set to 10 minutes or shorter from the viewpoint of suppressing the amount of liquid evaporation when using a 3-mL Petri dish as a container for accommodating the tryptophan-containing aqueous solution. In addition, the irradiation of oxygen radicals can be carried out by replenishing the solution as needed after irradiation of 10 minutes or shorter and further repeating irradiation of 10 minutes or shorter. In addition, the irradiation of oxygen radicals can also be performed as a continuous process, such as irradiating while circulating the solution through a liquid flow path, rather than as a batch process.

The liquid temperature of the tryptophan-containing aqueous solution before oxygen radical irradiation can be selected depending on the type of target tryptophan decomposition product, for example, from a range of the freezing point of the aqueous solution or higher and 40°C or lower. Specifically, when the primary objective is to generate FKYN or KYN as a tryptophan decomposition product, the above-described liquid temperature can be, from the viewpoint of suppressing the amount of liquid evaporation and the like, preferably 15°C or higher and 40°C or lower, and more preferably room temperature (24°C) or higher and 40°C or lower. When the primary objective is to generate Trp• as a tryptophan decomposition product and to facilitate its retention (details will be described below), the liquid temperature can be, preferably the freezing point of the aqueous solution or higher and room temperature (24°C) or lower, and more preferably the freezing point of the aqueous solution or higher and 15°C or lower.

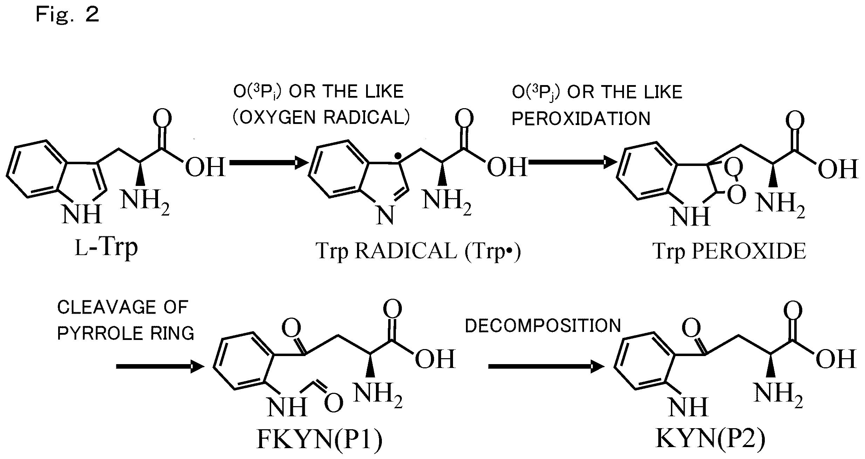

In the method for producing a tryptophan decomposition product of the present embodiment, as illustrated in Fig. 2, by irradiating a tryptophan-containing aqueous solution with oxygen radicals, a precursor including at least one of Trp• and Trp peroxide is generated from Trp, such as L-Trp, and a tryptophan decomposition product including at least one of FKYN and KYN is further generated from the precursor. As a result, in the method for producing a tryptophan decomposition product of the present embodiment, an oxygen radical-irradiated aqueous solution containing the tryptophan decomposition product is generated.

In addition, in the method for producing formylkynurenine of the present embodiment, as illustrated in Fig. 2, by irradiating a tryptophan-containing aqueous solution with oxygen radicals, a precursor including at least one of Trp• and Trp peroxide is generated from Trp, such as L-Trp, and at least FKYN is further generated from the precursor. As a result, in the method for producing formylkynurenine of the present embodiment, an oxygen radical-irradiated aqueous solution containing at least FKYN as a tryptophan decomposition product is generated.

In addition, in the method for producing kynurenine of the present embodiment, as illustrated in Fig. 2, by irradiating a tryptophan-containing aqueous solution with oxygen radicals, a precursor including at least one of Trp• and Trp peroxide is generated from Trp, such as L-Trp, and at least KYN is further generated from the precursor. As a result, in the method for producing kynurenine of the present embodiment, an oxygen radical-irradiated aqueous solution containing at least KYN as a tryptophan decomposition product is generated.

The precursor generated from Trp may be composed solely of Trp•, may be composed solely of Trp peroxide, or may include both Trp• and Trp peroxide. Highly active Trp• is produced by oxygen radical treatment of Trp but is a short-lived substance, as demonstrated in experimental examples described below. Trp• can be detected by electron spin resonance (ESR) spectroscopy. FKYN is generated by cleavage of a pyrrole ring in Trp peroxide. This cleavage occurs naturally in an aqueous solution. In other words, Trp peroxide decomposes on its own. Furthermore, since the generation of FKYN has been confirmed in the present disclosure, it is inferred that Trp peroxide is also generated, despite having a short lifespan. KYN is generated by the decomposition of FKYN.

A tryptophan-containing aqueous solution can be prepared (fixed) to be in any of acidic, neutral, or basic (alkaline) states. When the tryptophan-containing aqueous solution is prepared to be acidic, the amounts of FKYN and KYN, particularly KYN generated, in the oxygen radical-irradiated aqueous solution significantly increase, making it easier to increase the yield of FKYN and KYN as tryptophan decomposition products. In this case, the pH of the tryptophan-containing aqueous solution can be, preferably 1 or more and 3.5 or less, and more preferably 2 or more and 3.3 or less. In addition, to adjust the pH to an acidic range, the tryptophan-containing aqueous solution can be supplemented with, for example, citric acid (which may be referred to as Citric acid in the present specification and drawings), hydrochloric acid, nitric acid, or the like.

In addition, when the tryptophan-containing aqueous solution is prepared to be basic, the amount of KYN generated in the oxygen radical-irradiated aqueous solution significantly decreases, making it possible to mainly produce FKYN as a tryptophan decomposition product. In this case, the pH of the tryptophan-containing aqueous solution can be, preferably more than 7 and 10 or less, and more preferably 8 or more and 9 or less. In addition, to adjust the pH to a basic range, the tryptophan-containing aqueous solution can be supplemented with, for example, N-cyclohexyl-2-aminoethanesulfonic acid (which may be referred to as CHES in the present specification and drawings), sodium hydroxide, or the like.

In addition, when preparing the tryptophan-containing aqueous solution to be neutral, it can be supplemented with, for example, phosphoric acid (which may be referred to as PB in the present specification and drawings) or the like.

In the method for producing a tryptophan decomposition product of the present embodiment, the above-described tryptophan decomposition product is separated from the above-described oxygen radical-irradiated aqueous solution.

In addition, in the method for producing formylkynurenine of the present embodiment, FKYN is separated from the oxygen radical-irradiated aqueous solution.

In addition, in the method for producing kynurenine of the present embodiment, KYN is separated from the oxygen radical-irradiated aqueous solution.

The separation of the tryptophan decomposition product can be carried out, specifically, by fractionating the tryptophan decomposition product contained in the oxygen radical-irradiated aqueous solution and drying the obtained fractionated solution.

In addition, the separation of FKYN can be carried out, specifically, by fractionating FKYN contained in the oxygen radical-irradiated aqueous solution and drying the obtained fractionated solution.

In addition, the separation of KYN can be carried out, specifically, by fractionating KYN contained in the oxygen radical-irradiated aqueous solution and drying the obtained fractionated solution.

More specifically, the fractionation of each component contained in the oxygen radical-irradiated aqueous solution can be carried out by a liquid chromatograph (LC), a high-performance liquid chromatograph (HPLC), or the like. The fractionation of each component by LC or HPLC is preferably performed using gradient elution, which elutes each component while continuously varying the mobile phase composition.

As LC conditions and HPLC conditions, an ultraviolet absorption detector such as a photodiode array detector can be used as a detector for detecting components, and the measurement wavelength can be, for example, 200 nm to 300 nm. As a stationary phase (column), for example, "InertSustain AQ-C18" manufactured by GL Sciences Inc. can be used. As a mobile phase (gradient eluent), for example, ultrapure water/methanol or ultrapure water/acetonitrile can be used. The flow rate can be, for example, 0.1 mL/min to 10 mL/min.

In the method for producing a tryptophan decomposition product of the present embodiment, a first fractionated solution substantially free of KYN and containing FKYN may be fractionated, a second fractionated solution substantially free of FKYN and containing KYN may be fractionated, or both the first fractionated solution and the second fractionated solution may be fractionated. In addition, a third fractionated solution containing Trp• may be fractionated.

In addition, in the method for producing formylkynurenine of the present embodiment, at least the first fractionated solution substantially free of KYN and containing FKYN only needs to be fractionated. In addition, the second fractionated solution substantially free of FKYN and containing KYN may be fractionated, or both the first fractionated solution and the second fractionated solution may be fractionated. In addition, a third fractionated solution containing Trp• may be fractionated.

In addition, in the method for producing kynurenine of the present embodiment, at least the second fractionated solution substantially free of FKYN and containing KYN only needs to be fractionated. In addition, the first fractionated solution substantially free of KYN and containing FKYN may be fractionated, or both the second fractionated solution and the first fractionated solution may be fractionated. In addition, a third fractionated solution containing Trp• may be fractionated.

As a drying means, for example, an evaporator can be used. According to the evaporator, the eluent in the fractionated solution can be evaporated and removed to obtain FKYN or KYN as a powdered tryptophan decomposition product.

The method for producing a tryptophan decomposition product of the present embodiment has the above-described configuration. Therefore, according to the method for producing a tryptophan decomposition product of the present embodiment, by irradiating a tryptophan-containing aqueous solution that contains tryptophan with oxygen radicals, a tryptophan decomposition product such as formylkynurenine or kynurenine can be produced from the tryptophan simply in a relatively short time.

In addition, the method for producing formylkynurenine of the present embodiment has the above-described configuration. Therefore, according to the method for producing formylkynurenine of the present embodiment, by irradiating a tryptophan-containing aqueous solution that contains tryptophan with oxygen radicals, formylkynurenine, which is one of the tryptophan decomposition products, can be produced from tryptophan simply in a relatively short time.

In addition, the method for producing kynurenine of the present embodiment has the above-described configuration. Therefore, according to the method for producing kynurenine of the present embodiment, by irradiating a tryptophan-containing aqueous solution that contains tryptophan with oxygen radicals, kynurenine, which is one of the tryptophan decomposition products, can be produced from tryptophan simply in a relatively short time.

### (Second embodiment)

A method for producing a tryptophan radical-containing aqueous solution according to a second embodiment will be described. The method for producing a tryptophan radical-containing aqueous solution of the present embodiment is a method including: irradiating a tryptophan-containing aqueous solution that contains tryptophan with oxygen radicals to generate tryptophan radicals from the tryptophan; and generating a tryptophan radical-containing aqueous solution that contains the tryptophan radicals.

The details of the irradiation of the tryptophan-containing aqueous solution with oxygen radicals are as described in the first embodiment, and thus the explanation is not repeated. Trp• generated by the oxygen radical treatment of Trp has a bactericidal effect, as shown in the experimental examples described below. In other words, Trp• is a bactericidal substance.

The method for producing a tryptophan radical-containing aqueous solution of the present embodiment has the above-described configuration. Therefore, according to the above-described method for producing a tryptophan radical-containing aqueous solution, a tryptophan radical-containing aqueous solution that contains a tryptophan radical as a bactericidal substance and is suitably usable as a bactericidal aqueous solution can be produced.

As described above in the first embodiment, Trp• is a short-lived substance. However, based on the previous research results of the present inventors, it has been found that the lifespan of Trp• can be extended by maintaining a temperature of 15°C or lower.

Based on this finding, in the method for producing a tryptophan radical-containing aqueous solution of the present embodiment, the generated tryptophan radical-containing aqueous solution is preferably maintained in a liquid state at a liquid temperature of 15°C or lower. In this case, the disappearance of Trp• contained in the tryptophan radical-containing aqueous solution can be suppressed, and thus, it is possible to inhibit the reduction of the bactericidal effect when the tryptophan radical-containing aqueous solution is used as a bactericidal aqueous solution.

In addition, in the method for producing a tryptophan radical-containing aqueous solution of the present embodiment, the tryptophan radical-containing aqueous solution may be generated by irradiating the tryptophan-containing aqueous solution with the oxygen radicals while maintaining the tryptophan-containing aqueous solution in a liquid state at a liquid temperature of 15°C or lower, and the generated tryptophan radical-containing aqueous solution may be maintained in a liquid state at a liquid temperature of 15°C or lower. In this case as well, the disappearance of Trp• contained in the tryptophan radical-containing aqueous solution can be suppressed, and thus, it is possible to inhibit the reduction of the bactericidal effect when the tryptophan radical-containing aqueous solution is used as a bactericidal aqueous solution.

The liquid temperature is preferably 10°C or lower. In this case, the reduction of the bactericidal effect when the tryptophan radical-containing aqueous solution is used as a bactericidal aqueous solution can be further suppressed. In addition, the lower limit of the above-described liquid temperature is not particularly limited as long as it is higher than the freezing point of the aqueous solution.

The temperature adjustment of the liquid temperature of the tryptophan-containing aqueous solution and the tryptophan radical-containing aqueous solution can be achieved, for example, by providing a cooling function to a liquid holding unit equipped with a container capable of holding the liquid during oxygen radical irradiation.

The tryptophan radical-containing aqueous solution, as long as it contains at least Trp•, may also contain at least one selected from the group consisting of Trp peroxide, FKYN, and KYN.

The first embodiment can be appropriately referred to for other configurations and effects.

### (Third embodiment)

A bactericidal aqueous solution according to a third embodiment will be described. The bactericidal aqueous solution of the present embodiment includes a tryptophan radical-containing aqueous solution that contains a tryptophan radical.

The bactericidal aqueous solution of the present embodiment has the above-described configuration. Therefore, the bactericidal aqueous solution of the present embodiment can exhibit a bactericidal effect due to Trp•, which is a bactericidal substance.

The tryptophan radical-containing aqueous solution constituting the bactericidal aqueous solution of the present embodiment can be suitably produced, for example, by the method for producing a tryptophan radical-containing aqueous solution according to the second embodiment.

The bactericidal aqueous solution of the present embodiment is preferably maintained in a liquid state at a liquid temperature of 15°C or lower, more preferably at a liquid temperature of 10°C or lower. This configuration allows the suppression of the disappearance of Trp• and the inhibition of the reduction of the bactericidal effect. The lower limit of the above-described liquid temperature is not particularly limited as long as it is higher than the freezing point of the aqueous solution.

The bactericidal aqueous solution of the present embodiment can be suitably used, for example, for the sterilization of fungi or fungal organisms. The term "fungal organisms" in the present disclosure is used in a broad sense, including fungi.

Specific examples of fungi include green mold and black mold. Other examples of fungal organisms include Escherichia coli, Staphylococcus aureus, Salmonella, and methicillin-resistant Staphylococcus aureus (MRSA).

The bactericidal aqueous solution of the present embodiment can be suitably used, for example, for sterilization of crops, sterilization of soil, sterilization of hydroponic liquid media, and sterilization of medical instruments.

The first embodiment and the second embodiment can be appropriately referred to for other configurations and effects.

Hereinafter, the method for producing a tryptophan decomposition product, the method for producing formylkynurenine, the method for producing kynurenine, the method for producing a tryptophan radical-containing aqueous solution, and the bactericidal aqueous solution of the present embodiments will be described in more detail using the experimental examples.

### (Experimental Example 1)

3 mL of a tryptophan-containing aqueous solution containing 50 mM L-tryptophan and 200 mM phosphoric acid was placed in a Petri dish. Subsequently, this tryptophan-containing aqueous solution (pH = 6.3) was irradiated with oxygen radicals for 10 minutes using the radical source described in the first embodiment to obtain an oxygen radical-irradiated aqueous solution. At this time, the liquid temperature of the tryptophan-containing aqueous solution before oxygen radical irradiation was set to room temperature (24°C). A mixed gas of Ar gas and oxygen gas was used as a plasma generation gas supplied to the discharge unit of the radical source. The total gas flow rate of Ar gas and oxygen gas was 5 slm, the Ar gas flow rate was 4.97 slm, and the volume percentage of oxygen gas was 0.6%. The applied voltage in the discharge unit was 15 kV, and the frequency was 60 Hz. The irradiation distance of the oxygen radicals was set to 10 mm, and the density of triplet oxygen atoms (O(³Pⱼ)) was 2 × 10¹⁵ cm⁻³. The stage speed was 4 mm/s. The above-described tryptophan-containing aqueous solution not irradiated with oxygen radicals was designated as an oxygen radical-unirradiated aqueous solution.

Using HPLC (manufactured by Shimadzu Corporation, "Prominence/20A Series"), the components contained in the above-described oxygen radical-irradiated aqueous solution were fractionated. The fractionation HPLC conditions at this time were as follows.

### <Fractionation HPLC conditions>

Detector: Ultraviolet absorption detector
Measurement wavelength: 220 nm
Column: InertSustain AQ-C18 (particle size 5 µm, inner diameter 20 mm, length 150 mm) manufactured by GL Sciences Inc.
Mobile phase (gradient eluent): Ultrapure water (mobile phase A)/methanol (mobile phase B, hereinafter methanol may be denoted as MeOH)
Flow rate: 8 mL/min
Injection sample volume: 2 mL

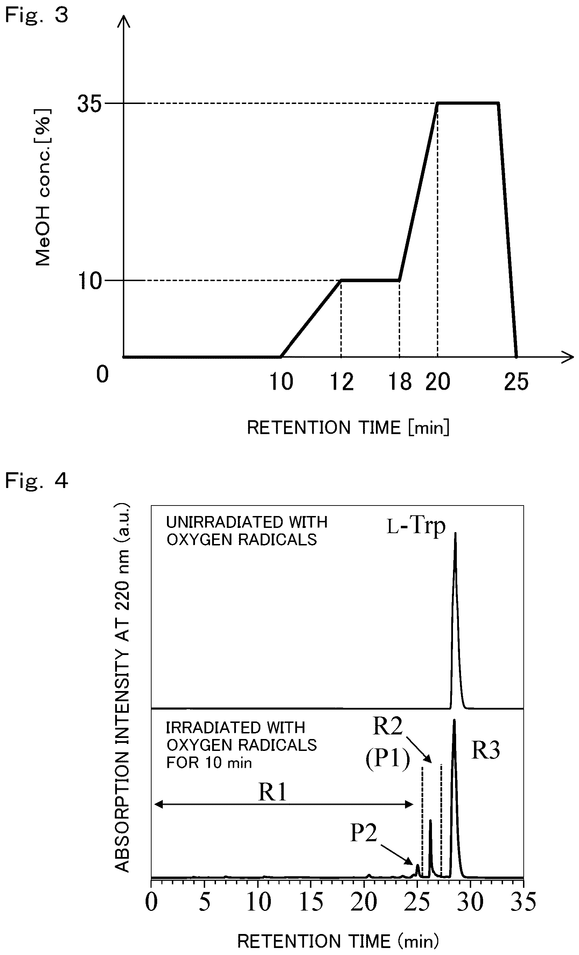

The gradient conditions of the mobile phase were set as shown in Fig. 3. In Fig. 3, the horizontal axis represents the retention time. The retention time refers to the duration for which the components in the oxygen radical-irradiated aqueous solution are retained and eluted by the column. In addition, the timing of injecting the oxygen radical-irradiated aqueous solution into the HPLC is set as a retention time of 0 min. In Fig. 3, the vertical axis represents the volume% of MeOH, which is the mobile phase B.

Fig. 4 shows the results of the above-described HPLC. As shown in Fig. 4, in the oxygen radical-unirradiated aqueous solution, a peak for L-Trp used as the raw material was detected, and no other peaks were observed. In contrast, in the oxygen radical-irradiated aqueous solution, peaks for a component P1 and a component P2 were observed in addition to the L-Trp peak. A fractionated solution R2 containing a fractionated component P1, corresponding to a retention time region R2, a fractionated solution R1 containing a fractionated component P2, corresponding to a retention time region R1, and a fractionated solution R3 containing fractionated L-Trp, corresponding to a retention time region R3, were each dried using an evaporator to obtain a powdery component P1 and component P2, and L-Trp. The L-Trp contained in the fractionated solution R3 is the undecomposed portion of the L-Trp used as the raw material. In this experimental example, each component was fractionated according to the corresponding retention time regions, but it is also possible to fractionate only the portions of the target components.

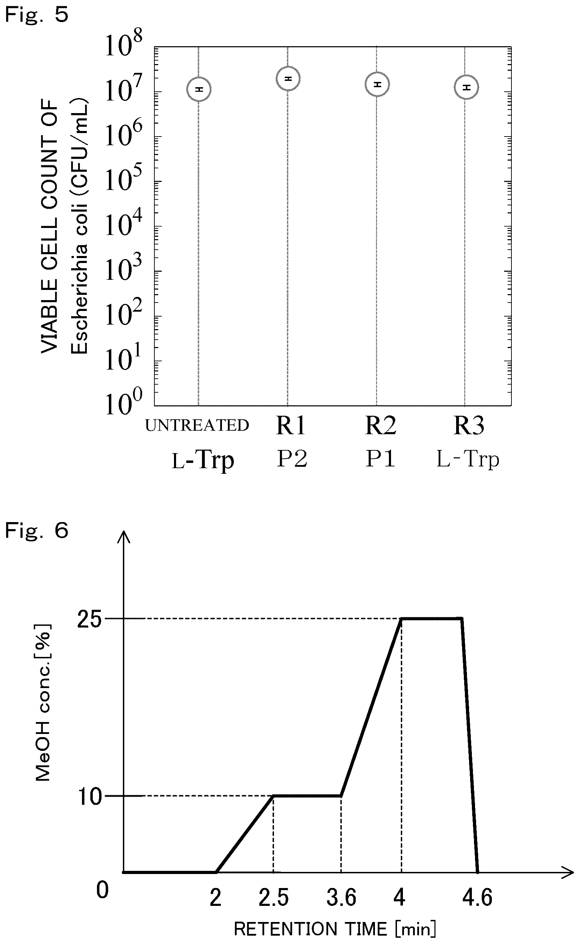

Each aqueous solution (liquid temperature: 24°C) prepared by redissolving each component in ultrapure water was mixed with a suspension of *Escherichia coli,* and after 10 minutes, the viable cell count of *Escherichia coli* was measured by a colony counting method. The viable cell count of *Escherichia coli* was also measured in the same manner for the oxygen radical-unirradiated aqueous solution. The results are shown in Fig. 5. As shown in Fig. 5, since none of the component P1 contained in the fractionated solution R2, the component P2 contained in the fractionated solution R1, and L-Trp contained in the fractionated solution R3 exhibits a bactericidal effect, it is understood that they are not bactericidal substances.

### (Experimental Example 2)

In almost the same manner as in Experimental Example 1, only the portions of the component P1 or the component P2 were fractionated, and the solvent was removed using an evaporator to obtain a powdery component P1 and component P2.

For the component P1 and the component P2, liquid chromatography/mass spectrometry (LC-MS) and nuclear magnetic resonance (NMR) analysis were conducted. The LC-MS conditions and NMR conditions were as follows, respectively.

### <LC-MS Conditions>

### -LC Conditions-

Detector: Ultraviolet absorption detector (photodiode array detector)
Measurement wavelength: 254 nm
Column: InertSustain AQ-C18 (particle size 5 µm, inner diameter 3 mm, length 30 mm) manufactured by GL Sciences Inc.
Mobile phase (gradient eluent): Ultrapure water (mobile phase A)/MeOH (mobile phase B)
The gradient conditions of the mobile phase were set as shown in Fig. 6.
Flow rate: 0.3 mL/min
Injection sample volume: 5 mL

### -MS conditions-

Device: "Exactive Plus" manufactured by Thermo Fisher Scientific Inc.
Ion source: ESI
Applied voltage: 3.5 kV
Polarity: Positive
Acquired MS range: 50 to 400 m/z

### <NMR Conditions>

Nuclear magnetic resonance analyzer: "Avance III HD" (600 MHz for ¹H, 151 MHz for ¹³C) manufactured by Bruker
Solvent: D₂O (99.9% for NMR spectroscopy, MERCK) with 0.05% acetonitrile (CH₃CN)
Number of integrations of ¹H: 32
Number of integrations of ¹³C: 4000

The acetonitrile mixed into the solvent serves as a calibration substance for a chemical shift on a horizontal axis of NMR. In theoretical NMR, the peak of a certain substance should always be detected at the same chemical shift value; however, the chemical shift value may slightly vary with each experiment depending on temperature, pH, and the like. Therefore, as a substance with a reported standard chemical shift value, acetonitrile (2.06 ppm in ¹H, 1.47 and 119•68 ppm in ¹³C) was mixed into the sample in this experimental example, and the chemical shift values of the NMR spectrum obtained in this experimental example were calibrated based on the acetonitrile peak.

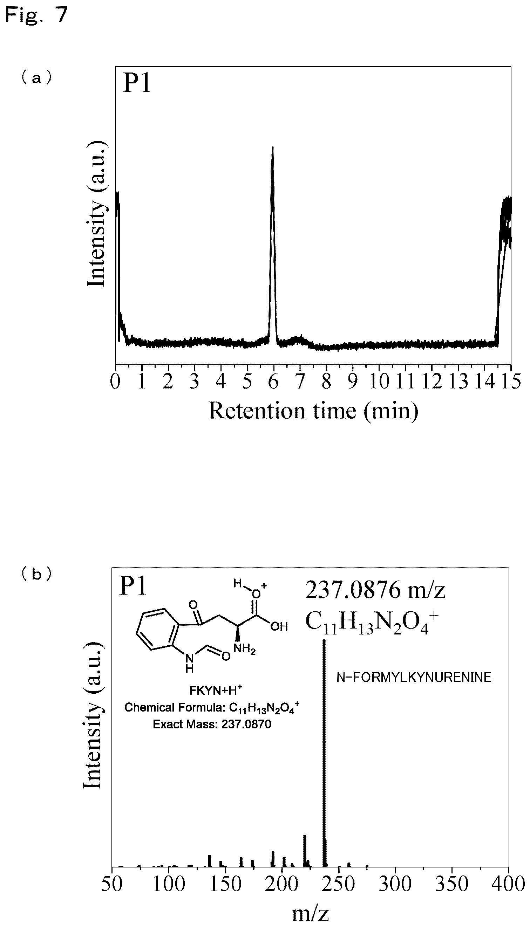

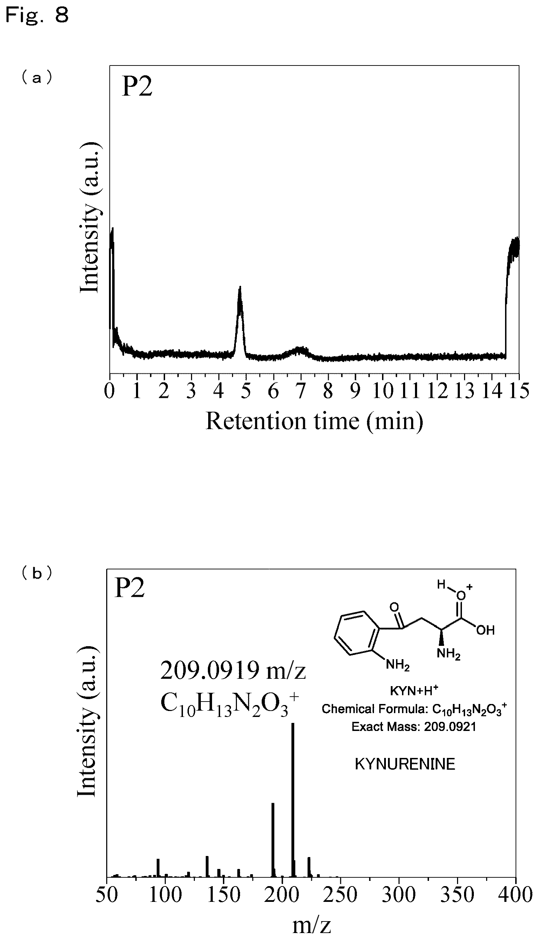

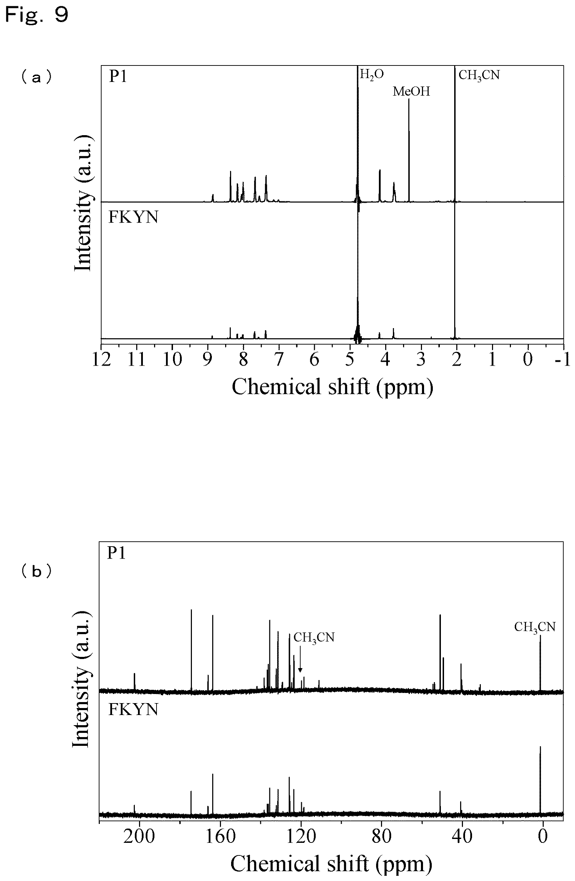

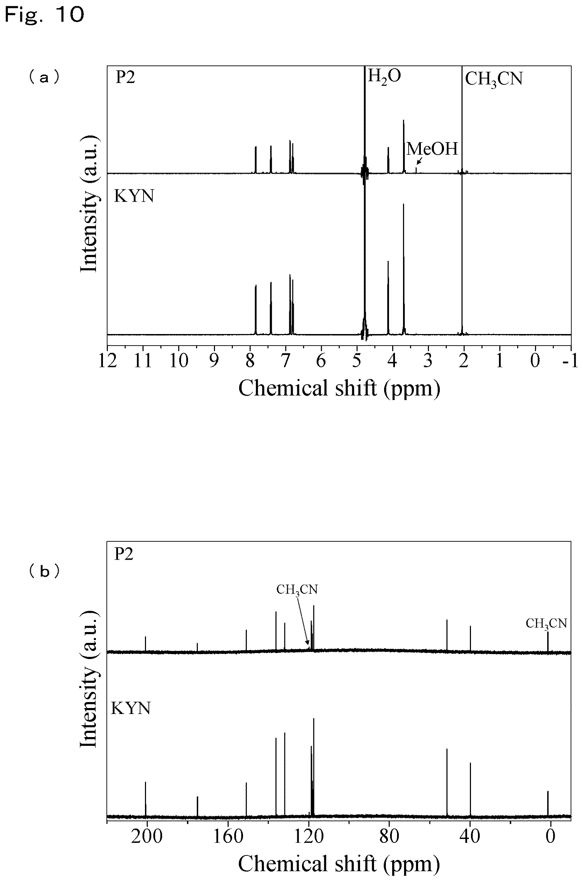

Fig. 7 shows an LC-MS chromatogram and an MS spectrum for the component P1. Fig. 8 shows an LC-MS chromatogram and an MS spectrum for the component P2. Fig. 9 shows (a) a ¹H-NMR spectrum and (b) a ¹³C-NMR spectrum for the component P1 and FKYN (standard substance). Fig. 10 shows (a) a ¹H-NMR spectrum and (b) a ¹³C-NMR spectrum for the component P2 and KYN (standard substance). According to Figs. 7 and 8, it was suggested that the component P1 is FKYN and the component P2 is FKN. According to Figs. 9 and 10, the spectrum of the component P1 matches the spectrum of the FKYN standard substance, and the spectrum of the component P2 matches the spectrum of the KYN standard substance. According to these results, it was confirmed that FKYN and FKN were finally generated as tryptophan decomposition products in the oxygen radical-irradiated aqueous solution obtained by irradiating the tryptophan-containing aqueous solution with oxygen radicals.

### (Experimental Example 3)

According to the results of Experimental Examples 1 and 2, since none of FKYN as the component P1 contained in the fractionated solution R2, KYN as the component P2 contained in the fractionated solution R1, and L-Trp contained in the fractionated solution R3 exhibits a bactericidal effect, they are not bactericidal substances. However, based on the previous research results of the present inventors, it is known that the oxygen radical-irradiated aqueous solution obtained by irradiating a tryptophan-containing aqueous solution with oxygen radicals has a bactericidal effect. From these findings, it is suggested that a short-lived bactericidal substance is generated by irradiating the tryptophan-containing aqueous solution with oxygen radicals.

Here, considering the generation pathway of FKYN and KYN by the irradiation of the tryptophan-containing aqueous solution with oxygen radicals as shown in Fig. 2, it can be said that it is highly likely that Trp• and Trp peroxide are generated as precursors from Trp in the process of generating FKYN and KYN, particularly suggesting that Trp• might be a bactericidal substance. Therefore, in this experimental example, an attempt was made to detect Trp• by electron spin resonance (ESR) spectroscopy.

Specifically, a tryptophan-containing aqueous solution containing 50 mM L-tryptophan and 10 mM DBNBS (3,5-dibromo-4-nitrosobenzenesulfonic acid sodium) as a spin trap agent was irradiated with oxygen radicals for 30 seconds in the same manner as in Experimental Example 1 to obtain an oxygen radical-irradiated aqueous solution (which may be referred to as 0.5 min wt DBNBS). The obtained oxygen radical-irradiated aqueous solution was injected into a quartz tube, and ESR measurement was conducted. Similarly, ESR measurement was also conducted on the tryptophan-containing aqueous solution containing the above-described DBNBS (hereinafter, it may be referred to as 0 min wt DBNBS) and an oxygen radical-irradiated aqueous solution obtained by irradiating a tryptophan-containing aqueous solution without the above-described DBNBS with oxygen radicals for 30 seconds (hereinafter, it may be referred to as 0.5 min wo DBNBS).

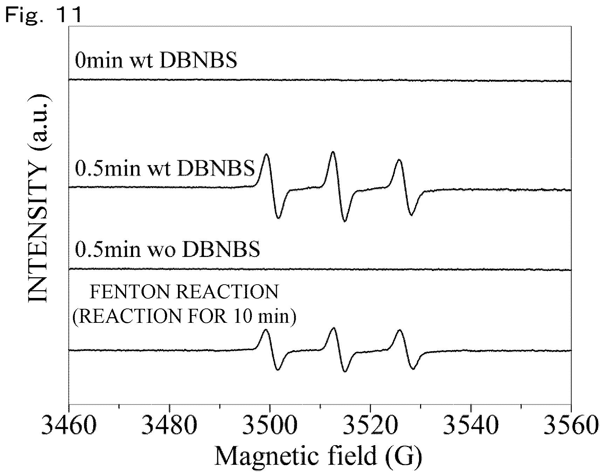

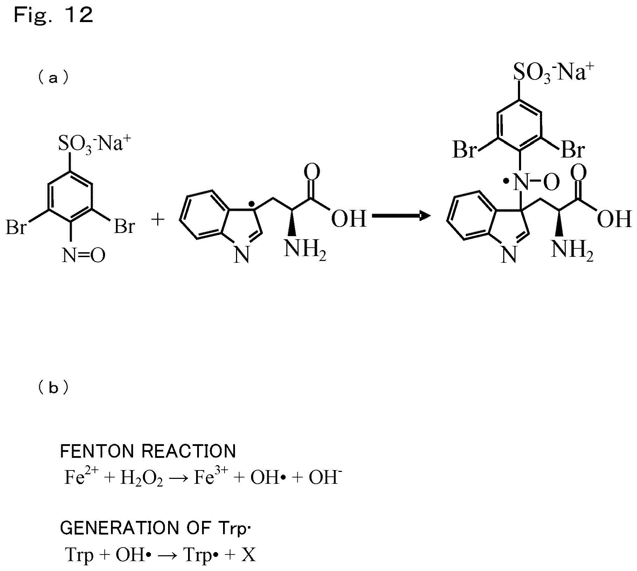

Fig. 11 shows the results of the above-described ESR measurement. In addition, Fig. 12 shows explanatory diagrams regarding (a) a reaction between DBNBS and Trp•, and (b) the Fenton reaction and a reaction between Trp and OH•. As shown in Fig. 11, for the oxygen radical-irradiated aqueous solution (0.5 min wt DBNBS) obtained by irradiating the tryptophan-containing aqueous solution containing DBNBS with oxygen radicals, three peaks were observed. These peaks are attributed to radicals (free radicals at the N atom site) derived from DBNBS that trapped spins of substances in the solution, as shown in Fig. 12(a). In contrast, for the oxygen radical-irradiated aqueous solution obtained by irradiating the tryptophan-containing aqueous solution without DBNBS with oxygen radicals (0.5 min wo DBNBS), the absence of DBNBS prevented the detection of the above-described three peaks. For the tryptophan-containing aqueous solution containing DBNBS (0 min wt DBNBS), the absence of spins to be trapped in the solution resulted in the above-described three peaks attributed to the radicals derived from the above-described DBNBS not being observed.

Here, as shown in Fig. 12 (b), it is known that Trp• is generated by the reaction between Trp and OH•. Therefore, the Fenton reaction shown in Fig. 12(b) was used to generate OH• in the oxygen radical-irradiated aqueous solution obtained by irradiating the tryptophan-containing aqueous solution without DBNBS with oxygen radicals (0.5 min wo DBNBS), and ESR measurement was conducted, and as a result, three peaks were observed at the same magnetic field as in the case of the oxygen radical-irradiated aqueous solution obtained by irradiating the tryptophan-containing aqueous solution containing DBNBS with oxygen radicals (0.5 min wt DBNBS). According to this result, it can be said that short-lived Trp• is generated as a bactericidal substance by the irradiation of the tryptophan-containing aqueous solution with oxygen radicals. In the formulae in Fig. 12(b), X is indicated because it is thought that OH extracts H from Trp and X becomes H₂O, but what is actually formed has not been accurately confirmed at this stage.

### (Summary of Experimental Examples 1 to 3)

From the above-described Experimental Examples 1 to 3, it was confirmed that the substances primarily generated by irradiating the tryptophan-containing aqueous solution with oxygen radicals are FKYN and KYN. In addition, Trp•, considered to be a precursor of FKYN and KYN, was detected by ESR measurement from the oxygen radical-irradiated aqueous solution. Based on the above, it is considered that short-lived precursors of FKYN and KYN (Trp• and Trp peroxide) are also expressed in large quantities by the oxygen radical irradiation in terms of total generation concentration, and it can be said that the above-described precursors are bactericidal substances.

### (Experimental Example 4)

3 mL of a tryptophan-containing aqueous solution containing 50 mM L-tryptophan and 200 mM phosphoric acid was placed in a Petri dish. Subsequently, this tryptophan-containing aqueous solution (pH = 6.3) was irradiated with oxygen radicals for a predetermined time using the radical source described in the first embodiment to obtain each oxygen radical-irradiated aqueous solution. In this experimental example, the irradiation times of the oxygen radicals were set to 1 min, 3 min, 5 min, 7 min, and 10 min. At this time, the liquid temperature of the tryptophan-containing aqueous solution before oxygen radical irradiation was set to room temperature (24°C). A mixed gas of Ar gas and oxygen gas was used as a plasma generation gas supplied to the discharge unit of the radical source. The total gas flow rate of Ar gas and oxygen gas was 5 slm, the Ar gas flow rate was 4.97 slm, and the volume percentage of oxygen gas was 0.6%. The applied voltage in the discharge unit was 15 kV, and the frequency was 60 Hz. The irradiation distance of the oxygen radicals was set to 10 mm, and the density of triplet oxygen atoms (O(³Pⱼ)) was 2 × 10¹⁵ cm⁻³. The stage speed was 4 mm/s. The above-described tryptophan-containing aqueous solution not irradiated with oxygen radicals (with an oxygen radical irradiation time of 0 min) was designated as an oxygen radical-unirradiated aqueous solution.

Using the above-described HPLC, the component P1 and the component P2 contained in the above-described oxygen radical-irradiated aqueous solution were fractionated, and after removing the solvent with an evaporator, quantification was performed with HPLC. The fractionation HPLC conditions at this time were as follows.

### <Analytical HPLC Conditions>

Detector: Ultraviolet absorption detector (photodiode array detector)
Measurement wavelength: 254 nm
Column: InertSustain AQ-C18 (particle size 5 µm, inner diameter 3 mm, length 30 mm) manufactured by GL Sciences Inc.
Mobile phase (gradient eluent): Ultrapure water (mobile phase A)/MeOH (mobile phase B)
The gradient conditions of the mobile phase were set as shown in Fig. 6.
Flow rate: 0.3 mL/min
Injection sample volume: 5 µL

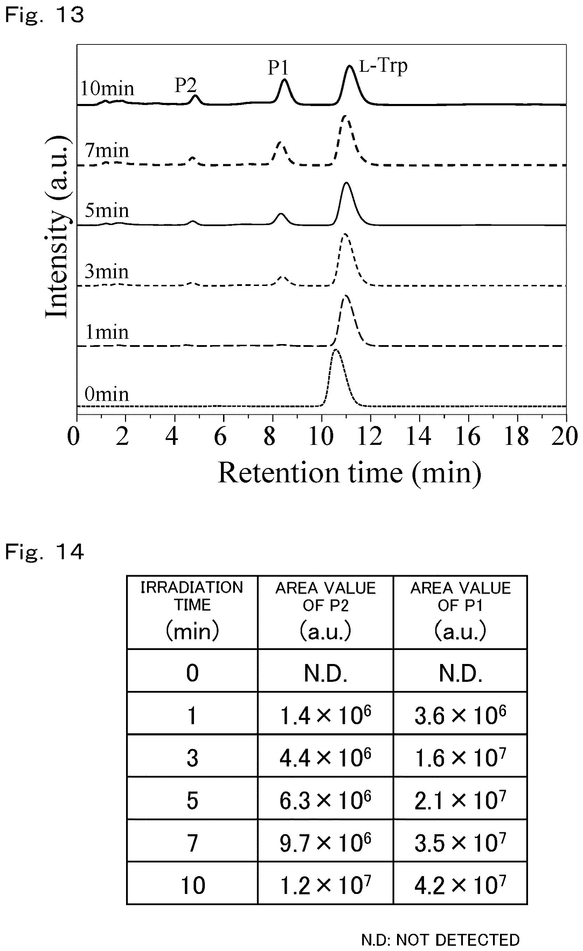

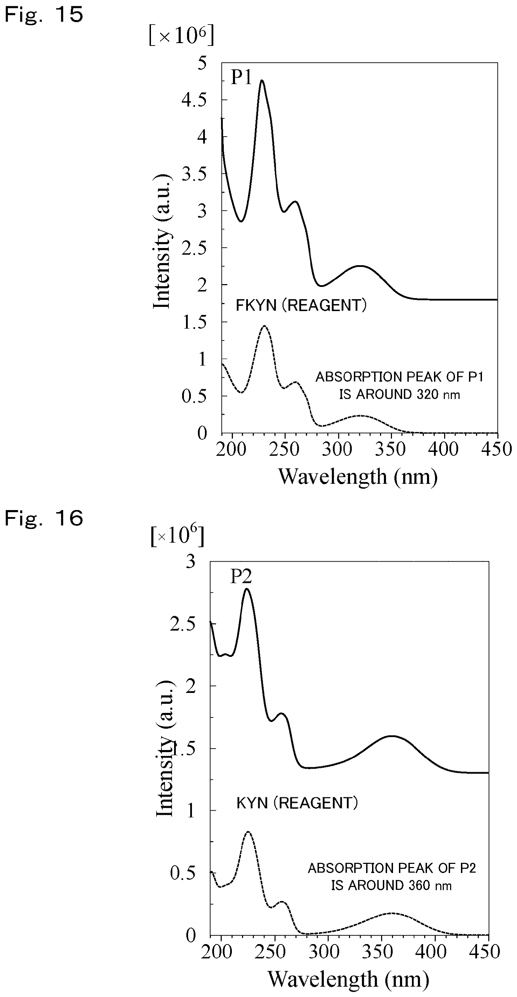

Fig. 13 shows chromatograms obtained by HPLC analysis. Fig. 14 collectively shows the relationship between the oxygen radical irradiation time and the area values of the components P1 and P2 calculated from the chromatograms in Fig. 13. Figs. 15 and 16 show UV absorption spectra of the components P1 and P2, respectively. According to Figs. 13 and 14, an increase in the area values of the components P1 and P2 in the oxygen radical-irradiated solution was confirmed with the increase in the oxygen radical irradiation time to the tryptophan-containing aqueous solution. In addition, according to Fig. 15, the absorption peak of the component P1 is around 320 nm and closely matches the absorption peak of the reagent FKYN. Similarly, according to Fig. 16, the absorption peak of the component P2 is around 360 nm and closely matches the absorption peak of the reagent KYN.

Therefore, according to this Experimental Example 4, it can be said that the increase in the irradiation time of oxygen radicals to the tryptophan-containing aqueous solution allows for an increase in the generation of FKYN and KYN in the oxygen radical-irradiated solution.

### (Experimental Example 5)

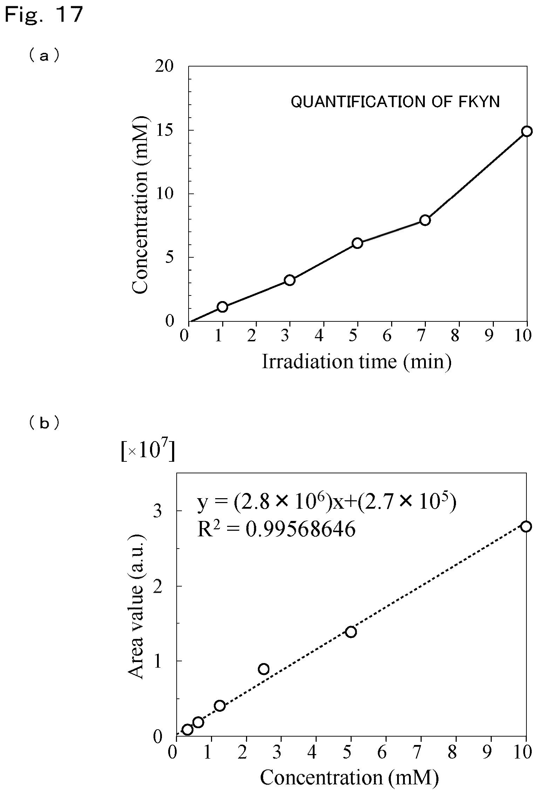

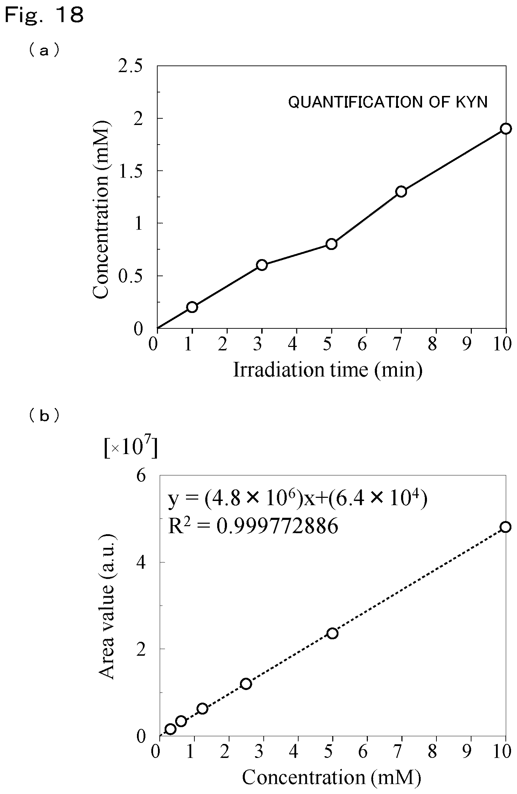

In the same manner as in Experimental Example 4, each oxygen radical-irradiated aqueous solution was prepared by irradiating the tryptophan-containing aqueous solution with oxygen radicals for a predetermined time. In addition, standard reagent solutions of FKYN and KYN were prepared. For each oxygen radical-irradiated aqueous solution, FKYN (component P1) and KYN (component P2) were fractionated using HPLC in the same manner as in Experimental Example 4, and the FKYN and KYN in the oxygen radical-irradiated aqueous solutions were quantified using the above-described standard reagent solutions. Fig. 17 shows the quantification results of FKYN. Specifically, Fig. 17(a) shows the concentration of FKYN determined from the peak area calculated from the chromatograms of FKYN with respect to the oxygen radical irradiation time and the calibration curve in Fig. 17(b), and Fig. 17(b) shows the calibration curve of the peak area calculated from the chromatograms of FKYN with respect to the standard solution concentration used to determine the above-described concentration. In addition, Fig. 18 shows the quantification results of KYN. Specifically, Fig. 18(a) shows the concentration of KYN determined from the peak area calculated from the chromatograms of KYN with respect to the oxygen radical irradiation time and the calibration curve in Fig. 18(b), and Fig. 18(b) shows the calibration curve of the peak area calculated from the chromatograms of KYN with respect to the standard solution concentration used to determine the above-described concentration.

According to Fig. 17, it can be seen that approximately 15 mM of FKYN can be fractionated when oxygen radicals are emitted for 10 min. Similarly, according to Fig. 18, it can be seen that approximately 2 mM of KYN can be fractionated when oxygen radicals are emitted for 10 min.

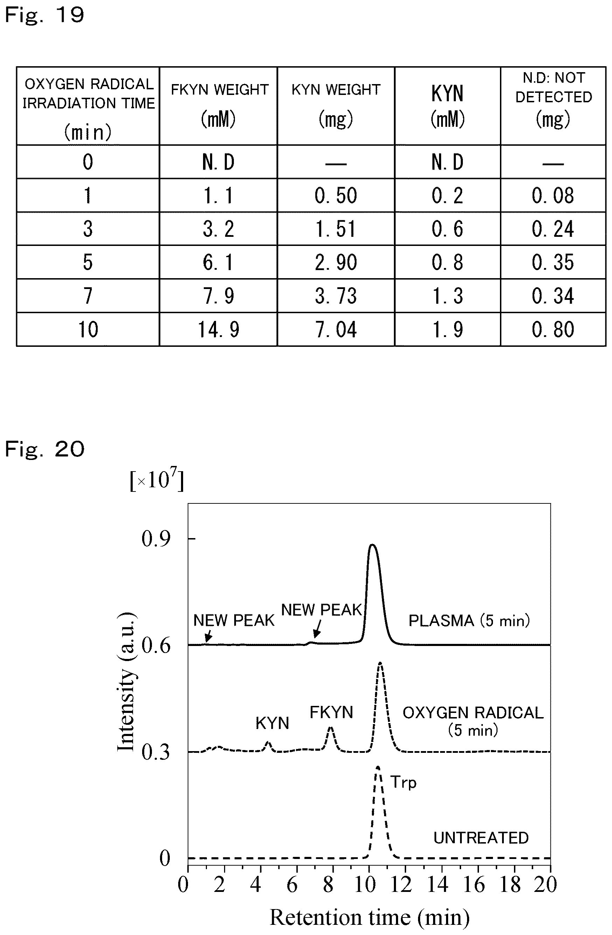

In addition, Fig. 19 collectively shows the relationship between the oxygen radical irradiation time and the amounts of fractionated FKYN and KYN. According to Fig. 19, it was confirmed that the substances primarily generated by irradiating the tryptophan-containing aqueous solution with oxygen radicals are FKYN and KYN. In addition, it was confirmed that 7 mg of FKYN and 0.8 mg of KYN can be purified with an oxygen radical irradiation time of 10 min, which is relatively short, for the tryptophan-containing aqueous solution.

### (Experimental Example 6)

In the same manner as in Experimental Example 4, an oxygen radical-irradiated aqueous solution was prepared by irradiating the tryptophan-containing aqueous solution with oxygen radicals. At this time, the irradiation time with oxygen radicals was set to 5 min. In addition, for comparison, a plasma-irradiated aqueous solution was prepared by irradiating a tryptophan-containing aqueous solution similar to that in Experimental Example 4 with non-equilibrium atmospheric pressure plasma using a plasma irradiation device with Ar gas. At this time, the plasma irradiation width was set to 3 mm × 20 mm. The plasma irradiation distance was set to 13 mm. The Ar gas flow rate was set to 2 slm. The plasma irradiation time was set to 0.5 to 10 min. The plasma irradiation device is configured to emit plasma generated at the discharge unit from the irradiation port, without having an intermediate unit or a curved path present in the radical source described above in the first embodiment.

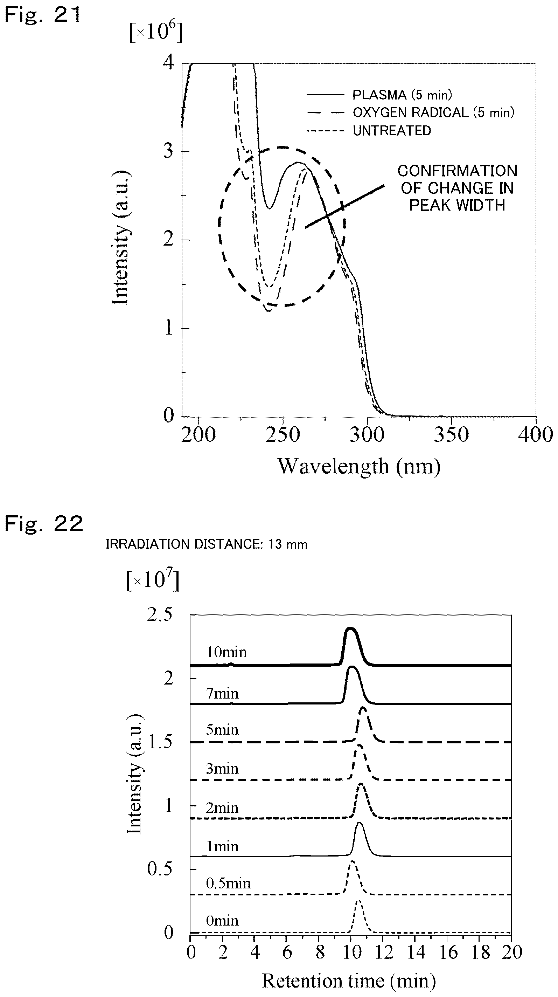

In the same manner as in Experimental Example 4, the components contained in the aqueous solution irradiated with oxygen radicals for 5 min, the aqueous solution irradiated with plasma for 5 min, and the tryptophan-containing aqueous solution were analyzed using HPLC under the above-described analytical HPLC conditions. Fig. 20 shows chromatograms obtained by HPLC analysis for the respective aqueous solutions. Fig. 21 shows UV absorption spectra of tryptophan obtained for the respective aqueous solutions. Fig. 22 shows chromatograms obtained by HPLC analysis of plasma-irradiated aqueous solutions with different plasma irradiation times. In Figs. 20 and 21, "oxygen radical (5 min)" refers to the above-described oxygen radical-irradiated aqueous solution with an oxygen radical irradiation time of 5 min, "plasma (5 min)" refers to the above-described plasma-irradiated aqueous solution with a plasma irradiation time of 5 min, and "untreated" refers to the above-described tryptophan-containing aqueous solution.

As shown in Figs. 20 and 21, FKYN and KYN were not detected in the conventionally known plasma irradiation. In addition, in the plasma treatment, the appearance of a small peak around a retention time of 1 min and an increase in the width of the tryptophan peak on the chromatogram were confirmed. In addition, as shown in Fig. 22, even when the plasma irradiation time in the conventionally known plasma irradiation was varied, KYN and KYN were not detected. This is considered to be due to the following reasons. That is, in the case of plasma irradiation, the density of triplet oxygen atoms (O(³Pⱼ)) is 0.5 × 10¹⁴ cm⁻³ at an irradiation distance of 13 mm, whereas in the case of oxygen radical irradiation using the radical source described above in the first embodiment, the density of triplet oxygen atoms (O(³Pⱼ)) is 2 × 10¹⁵ cm⁻³ at an irradiation distance of 10 mm. In addition, in oxygen radical irradiation, oxygen radicals are selectively emitted, whereas in plasma irradiation, Ar plasma is generated in the atmosphere, entraining the atmosphere and thus irradiating NO•, OH•, UV light, and the like. Compared to oxygen radical irradiation using a radical source, plasma irradiation results in a density of triplet oxygen atoms (O(³Pⱼ)) that is approximately 1/30, and therefore, it is considered that tryptophan decomposition products such as FKYN and KYN are not generated from Trp, and a different reaction proceeded.

### (Experimental Example 7)

A tryptophan-containing aqueous solution (pH = 6.3) containing 50 mM L-tryptophan and 200 mM phosphoric acid (PB), a tryptophan-containing aqueous solution (pH = 3.04) containing 50 mM L-tryptophan and 100 mM citric acid, and a tryptophan-containing aqueous solution (pH = 8.77) containing 50 mM L-tryptophan and 100 mM N-cyclohexyl-2-aminoethanesulfonic acid (CHES) were prepared.

In the same manner as in Experimental Example 4, an oxygen radical-irradiated aqueous solution (pH = 6.3) was prepared by irradiating the tryptophan-containing aqueous solution (pH = 6.3) with oxygen radicals. In addition, similarly, an oxygen radical-irradiated aqueous solution (pH = 3.04) was prepared by irradiating the tryptophan-containing aqueous solution (pH = 3.04) with oxygen radicals. In addition, similarly, an oxygen radical-irradiated aqueous solution (pH = 8.77) was prepared by irradiating the tryptophan-containing aqueous solution (pH = 8.77) with oxygen radicals. At this time, the irradiation time with oxygen radicals was set to 10 min for each. The above-described tryptophan-containing aqueous solution (pH = 6.3) not irradiated with oxygen radicals was designated as an oxygen radical-unirradiated aqueous solution (pH = 6.3).

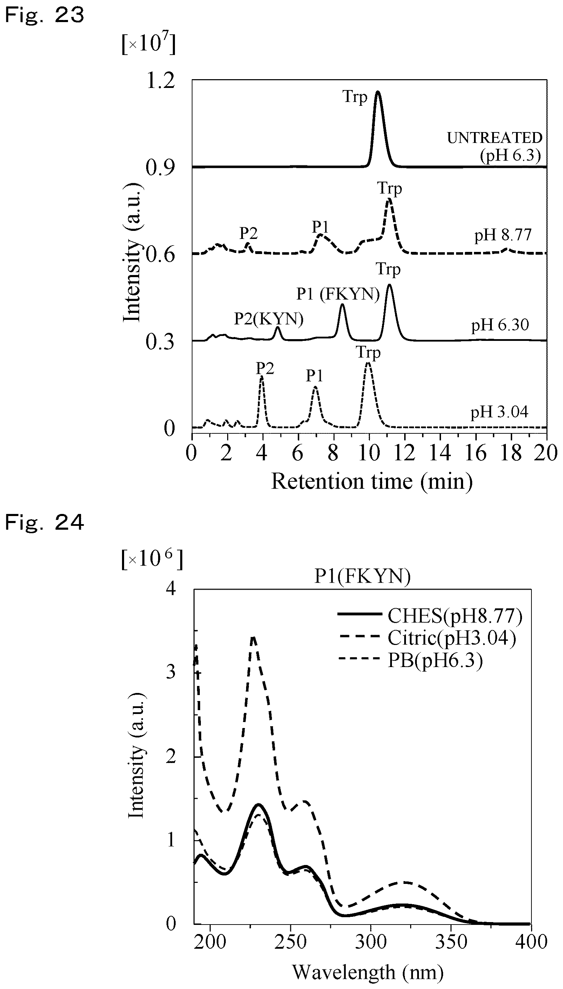

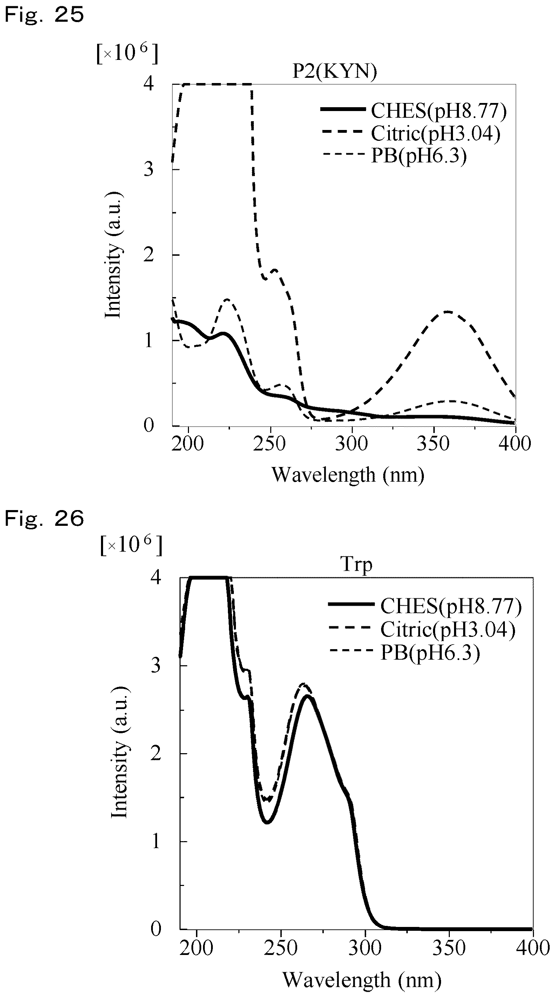

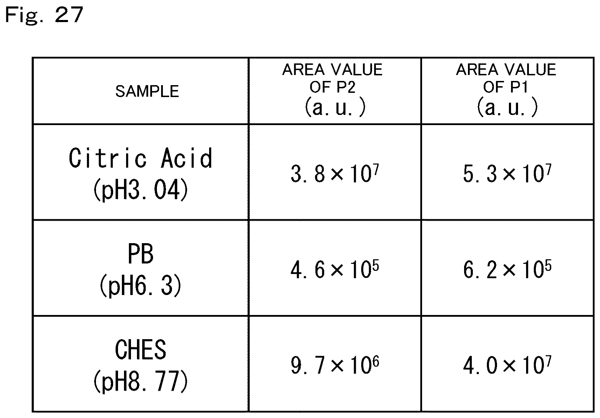

In the same manner as in Experimental Example 4, the components contained in the oxygen radical-irradiated aqueous solutions and the oxygen radical-unirradiated aqueous solution were analyzed using HPLC under the above-described analytical HPLC conditions. Fig. 23 shows chromatograms obtained by HPLC analysis for the respective aqueous solutions. Fig. 24 shows UV absorption spectra of the component P1 (FKYN) obtained for the respective aqueous solutions. Fig. 25 shows UV absorption spectra of the component P2 (KYN) obtained for the respective aqueous solutions. Fig. 26 shows UV absorption spectra of tryptophan (Trp). Fig. 27 shows area values of the component P1 (FKYN) and the component P2 (KYN) calculated from the chromatograms in Fig. 23 by HPLC analysis of the respective aqueous solutions.

According to Figs. 23 to 27, it can be seen that when the tryptophan-containing aqueous solution before oxygen radical irradiation is prepared to be basic, the amount of KYN generated in the oxygen radical-irradiated aqueous solution significantly decreases, and the generation shifts predominantly to FKYN. In addition, it was confirmed that when the tryptophan-containing aqueous solution before oxygen radical irradiation is prepared to be acidic, the amounts of FKYN and KYN generated, particularly the amount of KYN generated, in the oxygen radical-irradiated aqueous solution significantly increase, enabling a remarkable improvement in generation efficiency.

The present disclosure is not limited to the above-described embodiments and experimental examples, and various modifications are possible without departing from the gist thereof. In addition, the configurations shown in the embodiments and the experimental examples can be arbitrarily combined. In addition, the claims described in the claims at the time of filing the application can be arbitrarily combined with each another.

## Claims

1. A method for producing a tryptophan decomposition product, the method comprising:
obtaining an oxygen radical-irradiated aqueous solution that contains a tryptophan decomposition product by irradiating a tryptophan-containing aqueous solution that contains tryptophan with oxygen radicals so as to generate a precursor including at least one of a tryptophan radical and tryptophan peroxide from the tryptophan and thereafter generate a tryptophan decomposition product including at least one of formylkynurenine and kynurenine from the precursor; and
separating the tryptophan decomposition product from the oxygen radical-irradiated aqueous solution.

2. A method for producing formylkynurenine, the method comprising:
obtaining an oxygen radical-irradiated aqueous solution that contains formylkynurenine by irradiating a tryptophan-containing aqueous solution that contains tryptophan with oxygen radicals so as to generate a precursor including at least one of a tryptophan radical and tryptophan peroxide from the tryptophan and thereafter generate the formylkynurenine from the precursor; and
separating the formylkynurenine from the oxygen radical-irradiated aqueous solution.

3. A method for producing kynurenine, the method comprising:
obtaining an oxygen radical-irradiated aqueous solution that contains kynurenine by irradiating a tryptophan-containing aqueous solution that contains tryptophan with oxygen radicals so as to generate a precursor including at least one of a tryptophan radical and tryptophan peroxide from the tryptophan and thereafter generate the kynurenine from the precursor; and
separating the kynurenine from the oxygen radical-irradiated aqueous solution.

4. The method for producing a tryptophan decomposition product according to claim 1, wherein the tryptophan decomposition product contained in the oxygen radical-irradiated aqueous solution is fractionated to obtain a fractionated solution, and the obtained fractionated solution is dried to separate the tryptophan decomposition product.

5. The method for producing formylkynurenine according to claim 2, wherein the formylkynurenine contained in the oxygen radical-irradiated aqueous solution is fractionated to obtain a fractionated solution, and the obtained fractionated solution is dried to separate the formylkynurenine.

6. The method for producing kynurenine according to claim 3, wherein the kynurenine contained in the oxygen radical-irradiated aqueous solution is fractionated to obtain a fractionated solution, and the obtained fractionated solution is dried to separate the kynurenine.

7. The method for producing a tryptophan decomposition product according to claim 1 or claim 4, wherein the tryptophan-containing aqueous solution is a tryptophan-containing aqueous solution prepared to be acidic.

8. The method for producing a tryptophan decomposition product according to claim 1 or claim 4, wherein the tryptophan-containing aqueous solution is a tryptophan-containing aqueous solution prepared to be basic.

9. The method for producing formylkynurenine according to claim 2 or claim 5, wherein the tryptophan-containing aqueous solution is a tryptophan-containing aqueous solution prepared to be acidic.

10. The method for producing formylkynurenine according to claim 2 or claim 5, wherein the tryptophan-containing aqueous solution is a tryptophan-containing aqueous solution prepared to be basic.

11. The method for producing kynurenine according to claim 3 or claim 6, wherein the tryptophan-containing aqueous solution is a tryptophan-containing aqueous solution prepared to be acidic.

12. The method for producing kynurenine according to claim 3 or claim 6, wherein the tryptophan-containing aqueous solution is a tryptophan-containing aqueous solution prepared to be basic.

13. A method for producing a tryptophan radical-containing aqueous solution, wherein a tryptophan-containing aqueous solution that contains tryptophan is irradiated with oxygen radicals to generate tryptophan radicals from the tryptophan to thereby generate a tryptophan radical-containing aqueous solution that contains the tryptophan radicals.

14. The method for producing a tryptophan radical-containing aqueous solution according to claim 13, wherein the generated tryptophan radical-containing aqueous solution is maintained in a liquid state at a liquid temperature of 15°C or lower.

15. The method for producing a tryptophan radical-containing aqueous solution according to claim 13, wherein the tryptophan-containing aqueous solution is irradiated with the oxygen radicals while the tryptophan-containing aqueous solution is maintained in a liquid state at a liquid temperature of 15°C or lower to generate the tryptophan radical-containing aqueous solution; and the generated tryptophan radical-containing aqueous solution is maintained in a liquid state at a liquid temperature of 15°C or lower.

16. A bactericidal aqueous solution having a bactericidal effect and comprising a tryptophan radical-containing aqueous solution that contains tryptophan radicals.

17. The bactericidal aqueous solution according to claim 16, which is maintained in a liquid state at a liquid temperature of 15°C or lower.
